# EUROPEAN PATENT SPECIFICATION

(11) **EP 3 114 215 B1**
(45) Date of publication and mention of the grant of the patent: **14.04.2021**
(21) Application number: 15709149.7
(22) Date of filing: 09.03.2015
(51) Int. Cl.: A61P 35/00, A61P 35/02, C12N 5/0783, C12N 5/00

(54) **POOLED NK CELLS FROM OMBILICAL CORD BLOOD AND THEIR USES FOR THE TREATMENT OF CANCER AND CHRONIC INFECTIOUS DISEASE**
NK ZELLEN AUS GEMISCHTEN NABELSCHNURBLUT EINHEITEN UND IHRE VERWENDUNG ZUR BEHANDLUNG VON KREBS UND VON CHRONISCHEN ENTZÜNDUNGSERKRANKUNGEN.
CELLULES NK GROUPÉES DE SANG OMBILICAL ET LEUR UTILISATION POUR LE TRAITEMENT DU CANCER ET DE MALADIES CHRONIQUES INFECTIEUSES.

(30) Priority: 07.03.2014 EP 14305332
(43) Date of publication of application: 11.01.2017
(73) Proprietor: Emercell SAS, 34270 Saint-Mathieu-de-Tréviers (FR); Institut National de la Santé et de la Recherche Médicale, 75013 Paris (FR); Centre Hospitalier Universitaire de Montpellier, 34000 Montpellier (FR); UNIVERSITE DE MONTPELLIER, 34090 Montpellier (FR)
(72) Inventor: HENNO, Patrick, 34270 Saint Mathieu de Treviers (FR); VILLALBA GONZALEZ, Martin, 34070 Montpellier (FR); LU, Zhao Yang, 34830 Jacou (FR); ROSSI, Jean-François, 34990 Juvignac (FR)
(74) Representative: Touroude, Magali Linda
(86) International application number: PCT/EP2015/054837
(87) International publication number: WO 2015/132415

(56) References cited:
- WO-A2-2012/118349
- CA-A1- 2 703 428
- US-A1- 2012 121 544
- T-H JAING ET AL: "Transplantation of unrelated donor umbilical cord blood utilizing double-unit grafts for five teenagers with transfusion-dependent thalassemia", BONE MARROW TRANSPLANTATION, vol. 40, no. 4, 1 August 2007 (2007-08-01) , pages 307-311, XP055113119, ISSN: 0268-3369, DOI: 10.1038/sj.bmt.1705737
- S. AVERY ET AL: "Influence of infused cell dose and HLA match on engraftment after double-unit cord blood allografts", BLOOD, vol. 117, no. 12, 13 December 2010 (2010-12-13), pages 3277-3285, XP055113317, ISSN: 0006-4971, DOI: 10.1182/blood-2010-08-300491
- MEGHAN DELANEY ET AL: "High-resolution HLA matching in double-umbilical-cord-blood reduced-intensity transplantation in adults", TRANSFUSION, vol. 49, no. 5, 1 May 2009 (2009-05-01), pages 995-1002, XP055113366, ISSN: 0041-1132, DOI: 10.1111/j.1537-2995.2008.02077.x
- JUDITH A.E. SOMERS ET AL: "Double Umbilical Cord Blood Transplantation: A Study of Early Engraftment Kinetics in Leukocyte Subsets using HLA-Specific Monoclonal Antibodies", BIOLOGY OF BLOOD AND MARROW TRANSPLANTATION, vol. 19, no. 2, 1 February 2013 (2013-02-01), pages 266-273, XP055113361, ISSN: 1083-8791, DOI: 10.1016/j.bbmt.2012.09.022
- VELARDI A ET AL: "Natural killer cell allorecognition of missing self in allogeneic hematopoietic transplantation: a tool for immunotherapy of leukemia", CURRENT OPINION IN IMMUNOLOGY, ELSEVIER, OXFORD, GB, vol. 21, no. 5, 1 October 2009 (2009-10-01), pages 525-530, XP026682564, ISSN: 0952-7915, DOI: 10.1016/J.COI.2009.07.015 [retrieved on 2009-08-28]
- ANDREA VELARDI ET AL: "Clinical impact of natural killer cell reconstitution after allogeneic hematopoietic transplantation", SEMINARS IN IMMUNOPATHOLOGY, SPRINGER, BERLIN, DE, vol. 30, no. 4, 11 November 2008 (2008-11-11), pages 489-503, XP019655130, ISSN: 1863-2300, DOI: 10.1007/S00281-008-0136-1

## Description

The invention relates to the field of cell therapy, particularly NK cell mediated therapy. The present invention relates to a method of producing an ex vivo population of cells, preferably NK cells, from at least two umbilical cord blood units (UCB units), or fraction thereof containing said cells, by pooling said at least two UCB units to produce said population of cells. The present invention relates to the use of said cells, preferably NK cells, obtainable or obtained by the process according to the invention, as a composition for therapeutic use, preferably for the treatment of cancer and chronic infectious disease.

Natural Killer (NK) cells are a fundamental component of the innate immune system. They are capable of recognizing and destroying tumor cells as well as cells that have been infected by viruses or bacteria (Lanier LL, 2008; Nat Immunol 9: 495-502) Identification and characterization of NK cell receptors and their ligands over the last two decades have shed light on the molecular mechanisms of NK cell activation by tumor cells. The finding of inhibitory receptors supported the 'Missing self' hypothesis proposed by Karre whose pioneering work showed that NK cells killed tumor cells that lacked major histocompatibility complex (MHC) class-I molecule. The inhibitory receptors recognize MHC class I molecules whereas, the activating receptors recognize a wide variety of ligands (P. A. Mathew, J Cell Sci Ther, Volume 3, Issue 7).

NK cells are responsible of the graft versus leukemia (GvL) effect with minimal GvH (Graft versus Host) and HvG (Host versus Graft) effects, pointing attention to the development of immunotherapies involving NK cells. Data from several laboratories suggest that exploiting NK cell alloreactivity could have a large beneficial independently of NK cell source. Mismatched transplantation triggers alloreactivity mediated by NK cells, which is based upon "missing self recognition". Donor- versus-recipient NK cell alloreactions are generated between individuals who are mismatched for HLA-C allele groups, the HLA-Bw4 group and/or HLA-A3/11. KIR ligand mismatching is a prerequisite for NK cell alloreactivity because in 20 donor-recipient pairs that were not KIR ligand mismatched in the graft-versus-host direction, no donor alloreactive NK clones were found.

Another interesting point with NK cells is that even if NK cells also recognize the self-identity molecules (HLA molecules) mainly with their inhibitory receptors, they are activated through a complex equilibrium of activating signal and inhibiting signal and need the activating signal expressed only by infected, abnormal or tumoral cells to kill the cells. Then donor selection is easier because with NK cells alone donor and patient don't need to express quite exactly the same major HLA alleles (HLA match > 4/6 for total umbilical cord blood (UCB) graft for example). In contrast, NK expressing inhibitory receptors when the recipient doesn't express the corresponding HLA (absence of inhibitory signal = iKIR-HLA mismatch) lead to better tumor killing without leading to GvHD

Even if NK cells have a natural cytotoxic potential, their cytotoxic activity can be improved in vitro by different activation mechanisms, and most of these mechanisms are also able to amplify NK cells (with variable amplification factors) leading to more therapeutic cells, more efficient.

Finding a good way to amplify/activate NK cells is important to improve the therapeutic potential of these cells (quantity and potency).

In vitro activation protocols include cytokines and growth factor use, such as IL-2, IL-15, IL-18, IL-21, SCF, Flt3-L (...) with or without accessory cells such as peripheral blood mononuclear cells, tumoral cells or cell lines (see M. Villalba Gonzales et al., WO2009/141729). Using accessory cells presenting a particular iKIR-HLA mismatch (4 major iKIR-HLA mismatch : HLA A3/A11; HLA Bw4; HLA CI; HLA C2 and associated iKIR receptors).

Umbilical cord blood (UCB) has been shown to be a good source of NK cells, with higher NK cells percentages and good in vivo expansion/activation (see M. Villalba Gonzales et al, WO2012/146702).

Nevertheless, and despite the possibility to amplify and activate the NK cells contained in one UCB unit with a good rate of amplification, it is desirable to provide cell product, particularly NK cells product, for clinical therapies, available, purity, with high expansion rates and activation state and exhibiting for NK cells cytotoxic activity. In addition, it would be desirable that the method allows the production of a large quantity of cells, particularly activated NK cells, in a same batch (production lot), expected to treat at least more than 1, preferably, 50, more preferably around 100 patients, therapeutic agents needing to show less variability as possible.

To this end, it would be desirable to provide a method which offers the ability to obtain in a same lot of production, a large quantity of specific enriched cell populations, with a cell-manufacturing process which complies with the good manufacturing practice (cGMP), commercial- scale production and chemistry, manufacturing and controls standards of regulatory agencies.

This is the object of the present invention.

For the first time, and in a surprising manner, the Applicant succeeded in amplifying and pooling NK cells from different donors.

According to a first embodiment, the present invention relates to a method for the production of a population of expanded activated NK cells, comprising:
(A) producing a population of cells containing NK cells from at least n UCB units, or fraction thereof containing said NK cells, by a method comprising the steps of:
   (a) providing at least n umbilical cord blood units (UCB units), or fraction thereof containing said cells, with n ≥ 2, and
   (b) pooling said at least n UCB units, or fraction thereof containing said cells, to produce the population of cells
(B) expanding and activating said NK cells obtained from the step (A) in a suitable medium to produce said population of expanded activated NK cells; and
(C) recovering said expanded activated NK cells,
   wherein said method comprising in step (A),
   - a step (c) of depleting T cells from said population of cells obtained in (b), or
   - a step of depleting T cells contained in each of said n UCB units before the step (b) of pooling said UCB units,
wherein said n UCB units when pooled present the same pattern for major HLA class I groups genotype and wherein said major HLA class I group is selected from the group consisting of HLA A3/A11 which is recognized by KIR3DL2, HLA Bw4 which is recognized by KIR3DL1, HLA C group 1 which is recognized by KIR2DL2/3, and HLA C group 2 which is recognized by KIR2DL; and
wherein the suitable medium to expand and to activate the NK cells in step (B) comprising accessory cells, said accessory cells and the NK cells to be expanded and activated being HLA-KIR mismatched.

In a preferred embodiment, in the method of the present invention in step a), 3 ≤ n ≤ 50.

In another preferred embodiment, in the method according to the present invention, said accessory cells are irradiated.

In another preferred embodiment, in the method according to the present invention, said accessory cells are are immortalized.

In another preferred embodiment, in the method according to the present invention, each of said n UCB units or the pool of UCB units are red cells depleted.

Finally, the present invention is directed to the method according to the present invention wherein the UCB units used in step (A) are thawed UCB units from frozen stored UCB units.

In the present application, it is also disclosed a method of producing a population of cells, comprising the steps of:
(a) providing at least n umbilical cord blood units (UCB units), or fraction thereof containing said cells, with n ≥ 2, preferably 2 < n ≤ 100; and
(b) pooling said at least n UCB units, or fraction thereof containing said cells, to produce the population of cells.

In an embodiment, it is disclosed that 3 ≤ n ≤ 50, 3 ≤ n ≤ 25 being the most preferred.

In the context of the present invention, by "fraction of UCB unit containing said cells", it is intended to designate a fraction of the UCB unit containing at least the population of cells or part of said population which is desired to be produced.

In the present application, it is also disclosed a method, wherein said method further comprising the step of:
(c) depleting the T cells contained in the pool obtained in step (b), or
a step of depleting the T cells contained in each of the n UCB units before the step (b) of pooling.

The application further described method for the production of a population of expanded activated NK cells, wherein the n UCB units which are pooled in step b) present the same pattern for major HLA class I groups genotype.

In the present description, by "present the same pattern for major HLA class I groups genotype", it is intending to designate UCB units whose group of HLA molecules is recognized by the same inhibitory KIR or preferably wherein each HLA group present in the pooled n UCB is recognized by the same major inhibitory KIR by NK cells.

The application further described method, wherein each UCB present in the pooled n UCB belongs to a HLA group which is recognized by the same inhibitory KIR.

As used herein the term "KIR" or "inhibitory KIR" has its general meaning in the art and includes but is not limited to KIR2DL1, KIR2DL2, KIR2DL3, KIR3DL1 and KIR3DL2.

The main/major inhibitory KIRs are KIR2DL 1 , KIR2DL2, KIR2DL3 , KIR3DL 1 and KIR3DL2.

KIR2DL1 recognizes HLA-C w4 and related, 'group2' alleles. KIR2DL2 and KIR2DL3 recognize HLA-Cw3 and related, 'group 1 ' alleles. KIR3DL1 is the receptor for HLA-B allotypes with Bw4 motifs. Finally, KIR3DL2 is the receptor for HLA-A3/11.

The application further described a method for the production of a population of expanded activated NK cells, wherein said major HLA class I group is selected from the group consisting of HLA A3/A11 which is recognized by KIR3DL2, HLA Bw4, which recognized by KIR3DL1, HLA C group 1 which is recognized by KIR2DL2/3 and HLA C group 2 which is recognized by KIR2DL 1.

Source of UCB units are human UCB units, particularly frozen human UCB.

It is further described a method for producing an expanded population of cells from cells contained in n UCB units, comprising the step of:
(A) producing a population of cells from at least n UCB units, or fraction thereof containing said cells, by the method for producing a population of cells according to the present invention, optionally each UCB units has been preliminary and separately expanded for said cells before step A) ; and
(B) expanding the desired cells obtained from the population of cells obtained in step (A) in a suitable medium to produce said expanded population of desired cells.

In the method for producing an expanded population of cells from cells contained in n UCB units of the present invention, the step (B) can be an optionally step in case of each UCB units has been preliminary and separately expanded for said cells before the step b) of pooling in step A).

It is further described a method for producing a population of differentiated cells from desired cells contained in n UCB units, comprising the step of:
(A) producing a population of cells from said n UCB units, or fraction thereof containing said desired cells, by the method for producing a population of cells according to the present invention, optionally each UCB units has been preliminary and separately differentiated for said cells before step A); and
(B) differentiating the desired cells obtained from the preceding step in a suitable medium to produce said population of differentiated cells.

It is further described a method for producing a population of differentiated cells from cells contained in n UCB units, the step (B) of differentiating can be an optionally step in case of each UCB units has been preliminary and separately differentiated for said cells before the step b) of pooling in step A).

It is further described a method for producing a population of cells containing activated natural killer (NK) cells, comprising:
(A) producing a population of cells containing activated NK cells from at least n UCB units, or fraction thereof containing said NK cells, by the method for producing a population of cells according to the present invention, optionally each UCB units has been preliminary and separately expanded for said NK cells before step A);
(B) activating said NK cells obtained from the step (A) in a suitable medium to produce said population of cells containing activated NK cells;
C) optionally, recovering said activated NK cells from said population.

It is further described a method for producing a population of expanded activated NK cells, comprising:
(A) producing a population of cells containing NK cells from at least n UCB units, or fraction thereof containing said NK cells, by the method for producing a population of cells according to the present invention, optionally each UCB units has been preliminary and separately expanded and activated for said NK cells before step A);
(B) expanding and activating said NK cells obtained from the step (A) in a suitable medium to produce said population of expanded activated NK cells; and
(C) optionally, recovering said expanded activated NK cells.

It is further described a method for producing a population of expanded, optionally, activated NK cells from n UCB units, said method comprising the step of:
i) providing at least n UCB units, or fraction thereof containing NK cells, with n > 2, preferably 2 < n < 100 or 3 < n < 50, more preferably 3 < n < 25, and wherein said at least n UCB units present the same pattern for major HLA class I groups genotype, preferably wherein each HLA group present in the pooled n UCB is recognized by the same major inhibitory KIR by NK cells;
ii) optionally red cell-/erythrocytes-depleting each UCB unit, preferably by density gradient separation, more preferably by Ficoll-Paque® density gradient separation, by the Hetastarch (Hydroxyethyl Starch; HES) method , by using the PrepaCyte® CB device or by a step of freezing and thawing;
iii) optionally, the population of cells obtained in step i) or ii) is frozen, kept in liquid nitrogen and thawed before step iv);
iv) depleting the T cells contained in each UCB unit;
v) for each of the UCB units obtained in the preceding step, separately expand and, optionally, activate the NK cells contained in one UCB unit by contacting the NK cells contained in the UCB unit, or fraction thereof containing NK cells, in a suitable medium to produce said expanded population and, optionally, activated NK cells for each UCB unit, preferably during 3 to 28 days;
vi) pooling the n UCB units cells obtained in the preceding step UCB units, or fraction thereof containing NK cells, to produce a population of pooled expanded and, optionally, activated NK cells.

It is further described a method for producing a population of expanded and, optionally, activated NK cells from n UCB units, said method comprising the step of:
i) providing at least n UCB units, or fraction thereof containing NK cells, with n ≥ 2, preferably 2 < n ≤ 100 or 3 ≤ n ≤ 50, more preferably 3 ≤ n ≤ 25, and wherein said at least n UCB units present the same pattern for major HLA class I groups genotype, preferably wherein each HLA group present in the pooled n UCB is recognized by the same major inhibitory KIR by NK cells;
ii) optionally red cell-/erythrocytes-depleting each UCB unit, preferably by density gradient separation, more preferably by Ficoll-Paque® density gradient separation (type Ficoll-Paque PREMIUM®), by the Hetastarch (Hydroxyethyl Starch; HES) method, by using the PrepaCyte® CB device or by a step of freezing and thawing; iii) optionally, the population of cells obtained in step i) or ii) is frozen, kept in liquid nitrogen and thawed before step iv);
iv) for each of the UCB units obtained in the preceding step, separately expand and, optionally, activate the NK cells contained in one UCB unit by contacting the NK cells contained in the UCB unit, or fraction thereof containing NK cells, in a suitable medium to produce said expanded population and, optionally, activated NK cells for each UCB unit, preferably during 3 to 28 days;
v) pooling the nUCB units cells obtained in the preceding step UCB units, or fraction thereof containing NK cells, to produce a population of pooled expanded and, optionally, activated NK cells; and
vi) optionally, depleting the T cells contained in the pooled NK cells obtained after step v).

It is disclosed that depleting the T cells contained in the pooled NK cells obtained after step v) is not an optionally step and is part of the method.

It is described that in step vi) of depleting the T cells contained in the pooled NK cells obtained after step v) is followed by a step of selecting the NK cells exhibiting the CD56+ bio marker, whether it is still desirable to eliminate remaining non-activated NK cells at this end of the process.

It is further described a method for producing a population of expanded and, optionally, activated NK cells from n UCB units, said method comprising the step of:
i) providing at least n UCB units, or fraction thereof containing NK cells, with n ≥ 2, preferably 2 < n ≤ 100 or 3 ≤ n ≤ 50, more preferably 3 ≤ n ≤ 25, and wherein said at least n UCB units present the same pattern for major HLA class I groups genotype, preferably wherein each HLA group present in the pooled n UCB is recognized by the same major inhibitory KIR by NK cells;
ii) optionally red cell-/erythrocytes-depleting each UCB unit, preferably by density gradient separation, more preferably by Ficoll-Paque® density gradient separation, by the Hetastarch (Hydroxyethyl Starch; HES) method, by using the PrepaCyte® CB device or by a step of freezing and thawing;
iii) optionally, the population of cells obtained in step i) or ii) is frozen, kept in liquid nitrogen and thawed before step iv);
iv) optionally, or preferably, depleting the T cells contained in each UCB unit;
v) pooling the nUCB units cells obtained in the preceding step UCB units, or fraction thereof containing NK cells, to produce a population of pooled NK cells; and
vi) expanding and, optionally, activating the pooled NK cells obtained in the preceding step by contacting the NK cells contained in the pool, or fraction thereof containing NK cells, in a suitable medium to produce said population of pooled expanded and, optionally, activated NK cells, preferably during 1 to 5 weeks, preferably the amplification factor for NK cells after the expanding step(s) is at least 100, preferably, 200, 300 or 500 for an expanding/activation step(s) total duration comprised between 9 and 28 days.

It is further described a method for producing a population of expanded, and, optionally, activated NK cells from n UCB units, said method comprising the step of:
i) providing at least n UCB units, or fraction thereof containing NK cells, with n ≥ 2, preferably 2 < n ≤ 100 or 3 ≤ n ≤ 50, more preferably 3 ≤ n ≤ 25, and wherein said at least n UCB units present the same pattern for major HLA class I groups genotype, preferably wherein each HLA group present in the pooled n UCB is recognized by the same major inhibitory KIR by NK cells;
ii) optionally red cell-/erythrocytes-depleting each UCB unit, preferably by density gradient separation, more preferably by Ficoll-Paque® density gradient separation, by the Hetastarch (Hydroxyethyl Starch; HES) method, by using the PrepaCyte® CB device or by a step of freezing and thawing;
iii) optionally, the population of cells obtained in step i) or ii) is frozen, kept in liquid nitrogen and thawed before step iv);
iv) pooling the nUCB units cells obtained in the preceding step UCB units, or fraction thereof containing NK cells, to produce a population of pooled NK cells;
v) optionally, or preferably depleting the T cells contained in the pooled NK cells obtained after step iv; and
vi) expanding and, optionally, activating the pooled NK cells obtained in the preceding step by contacting the NK cells contained in the pool, or fraction thereof containing NK cells, in a suitable medium to produce said population of pooled expanded and, optionally, activated NK cells, preferably during 1 to 5 weeks, preferably the amplification factor for NK cells after the expanding step(s) is at least 100, preferably, 200, 300 or 500 for an expanding/activation step(s) total duration comprised between 9 and 28 days

All the methods according to the present invention and relative to the production of activated/ expanded NK cells are particularly suitable for preparing activated NK cells, from pooled UCB units, with miss expression of one of the following KIRs: KIR2DL2 and KIR2DL3, KIR2DL1, KIR3DL1 and KIR3DL2. Consequently, in this case, the activated/expanded pooled NK cells as above prepared according to the present invention will be alloreactive toward cells from others which lack the corresponding KIR ligand and, conversely, will be tolerant of cells from another individual who has the same KIR ligands.

Thus, by the method of the present invention, it can be produced a collection, or a therapeutic cells bank, of at least 2 different production lots, preferably 3, more preferably 4, of pooled activated/expanded NK-cells obtainable by a method for producing NK cells of the invention, or a collection of at least 2, 3 or 4 fractions of said production lots, and wherein each production lot exhibits a different miss expression of one of the major inhibitory KIRs, preferably selected from the group of KIR2DL2 and KIR2DL3, KIR2DL1, KIR3DL1 and KIR3DL2 inhibitory KIRs.

It is further disclosed such a collection of at least 2 different production lots, preferably 3, more preferably 4, of pooled activated/expanded NK-cells obtainable by a method for producing pooled activated/expanded NK-cells NK cells of the invention is comprised in the present invention.

It is further described a collection of storage containers comprises at least 2, 3 or 4 containers that each contains a pooled activated/expanded NK-cells, or fraction thereof, obtainable by a method for producing NK cells of the invention and exhibiting a particular miss expression of one of the major inhibitory KIRs.

It is further described one production lot, or fraction thereof which is needed in quantity for treating one patient, of the claimed collection can be used for transplantation in a patient in need thereof, preferably a patient exhibiting target cells that do not express the specific major KIR ligand which is recognized by the pooled activated/amplified NK cells production lot which will be transplanted.

HLA/KIRs genotyping/phenotyping of UCB/NK cells or patient target cells may be performed by any well-known standards methods.

It is further described a method for producing a population of expanded, and, activated NK cells from n UCB units, wherein said suitable medium suitable to expand and to activate the NK cells comprised accessory cells and/or at least one suitable NK activated factor.

It is further described a method for producing a population of expanded, and activated NK cells from n UCB units, wherein said accessory cells are selected from the group of:
- mammals cells, preferably human cell, more preferably from HLA-typed collection of cells and, optionally, irradiated cells, particularly gamma-, X- or UV-irradiated cells, gamma- irradiated cells being preferred;
- transformed mammals cells, preferably human cells, wherein in said cell, the expression of one gene encoding for a Killer-Cell Immunoglobulin-like Receptor(s) (KIR) ligand has been inhibited.

It is further described a method for producing a population of expanded, and activated NK cells from n UCB units, wherein said cells from HLA-typed collection of cells are from the PLH cell line, preferably selected from the group of ECACC N°. 88052047, IHW number 9047 and HOM-2 , ID n°HC107505, IHW number 9005.

It is further described a method for producing a population of expanded and activated NK cells from n UCB units, wherein said accessory cell is a transformed mammal cell wherein the expression of one gene encoding for a KIR ligand has been inhibited and which further comprises the inhibition or the reduction of the MHC-I expression and/or the inhibition of the expression of the ERK5 gene. The method for preparing such accessory cells is well known by the skilled person (see WO 2012/146702 published on November 1, 2012.

The inhibition or reduction of the MHC-I expression is said accessory cell may be performed by any method well known in the art. For example said methods are exemplified in the international patent application publication WO2009141729A2. Typically, said inhibition or reduction of MHC-I expression is performed by using inhibitor of beta-2-microglobulin gene expression.

As indicated above, said accessory cell will be presenting a negative ERK5 phenotype. The term "cell presenting a negative ERK5 phenotvpe" means a cell having a reduction of at least 10%, preferably 25% to 90%, for example 25% to 50% or 50% to 75% in the level of expression or the quantity of ER 5 protein present in the cell, in particular in the mitochondrial fraction, compared with its level of expression.

The inhibition or reduction of the ER 5 gene expression is said cell may be performed by any method well known in the art. For example said methods are exemplified in the international patent application publication WO2009141729A2. Typically, said inhibition or reduction of gene ER 5 expression is performed by using inhibitor of ER 5 gene expression.

It is further described a method for producing a population of expanded and activated NK cells from n UCB units, wherein said accessory cells have been immortalized, preferably by Epstein Barr Virus (EBV) transformation.

As a result said accessory cell will constitute a cell line that proliferate indefinitely in culture. Methods for immortalizing cells are well known in the art, particularly using the "Epstein Barr virus" ("EBV") process for immortalize human lymphocyte.

It is further described a method for producing a population of expanded and activated NK cells from n UCB units, wherein said suitable medium comprised as suitable NK activated factor interleukin-2 (IL-2), IL-7,and/or IL-12 and/or IL-15, or with alpha- or beta- interferon, preferably human recombinant activated factor.

When accessory cells are not used for activating the NK cells, the activation can be carried out using the following possible medium containing NK cells activating factor :
1/ IL-2 5 ng/ml +/- anti-CD3 50 ng/ml + IL-7 10 ng/ml + IL-12 10 ng/ml, preferably after 7 days ;
21 hIL-15 30ng/ml + hIL-21 30 ng/ml (PeproTech) + hydrocortisone 10<"6> M > CD34+ 21 days of cultivation thus 21 days of cultivation for the maturation/activation of the NK Cells;
3/ IL-2 500U/ml + beads CD335 (NKp46) and CD2 ;
4/ Mix of cytokines IL-7, SCF, IL-2, IL-15 (strong concentration) and GM-CSF, G-CSF, IL-6 (low concentration) for NK cells expansion from D14 to D42, in bioreactor,fromCD34+ amplification; DO-9= low molecular heparin + mix of cytokines (strong concentration) SCF, Flt3L, TPO, IL-7 and (low concentration) GM-CSF, G-CSF, IL-6 (CD34+ amplification); J9-14 = low molecular heparin + mix of cytokines (strong concentration) SCF, Flt3L, IL-15, IL-7 and GM-CSF, G-CSF, IL-6 (low concentration ,NK differentiation). (IL-18 and IFN alpha can be also used).

- Activation and expanding in presence of accessory cells :
1/ IL-2 500U/ml + autologous/allogenic irradiated feeder PBMC (25 Gy) ou EBV-LCL (1OOGy), ratio feeder cells:NK 20: 1 (or 10: lfor UCB unit scale-up ) at DO
21 IL-2 200U/ml + mytomycin treated feeder (PBMC+K562 ratios 1 : 1) ratio feeder cells:NK 8: 1
3/ IL-2 500U/ml + allogenic irradiated feeder PBMC (5 000 rad) at DO abd D7 ratio feeder cells:total 10: 1 + OKT3 (anti-CD3) 30ng/ml in the culture medium or preincubated with feeder cells
4/ IL-2 500U/ml + irradiated feeder Jurkat-KLl (300Gy) at DO
5/ IL-2 500U/ml + autologous irradiated feeder PBMC (2000 rad, + OKT3 lOng/ml at the beginning for stimulate the T lymphocytes of the feeders cells (depleted din the non- irradiated faction)) ratio feeder cells :NK 5 : 1 JO
6/ IL-2 + IL-15 + feeder irradiated feeder K562-mbl5-41BBL (1OOGy)

It is further described a method for producing a population of expanded and activated NK cells from n UCB units, wherein the step of depleting the T cells is carried out by a method comprising the step of:
- contacting the cells with a depleting antibody; and
- removing the cells detected by said depleting antibody.

The depleting antibody can be at least an antibody selected from the group consisting of an anti-CD3, an anti-CD 14, and an anti-CD 20 antibody, preferably an anti-CD3 antibody.

In the population of depleted cells obtained, less than 0.5 % or even less than 0.1 % or even less than 0.001 % are CD3 positive cells.

It is also described a method for producing a population of expanded and activated NK cells from n UCB units, wherein each UCB unit or the pooled n UCB units are red cell-/ erythrocytes depleted, preferably by density gradient separation, more preferably by Ficoll-Paque® density gradient separation, by the Hetastarch (Hydroxyethyl Starch; HES) method, by using the PrepaCyte® CB device or by a step of freezing and thawing.

It is further described a method for producing a population of expanded and activated NK cells from n UCB units, wherein each UCB unit or the pooled n UCB units are red cell-depleted by a method comprising the lysis of the red blood cells, particularly by a method including a step of freezing and thawing the cells contained in each of the UCB unit or in the n UCB units pooled cells.

It is also described a method for producing a population of expanded and activated NK cells from n UCB units, wherein the UCB units used in step b) or in step i) are thawed UCB units from frozen stored UCB units.

It is further described a method for producing a population of expanded and activated NK cells from n UCB units, wherein the UCB units used in step b) or in step i) are thawed UCB units from frozen stored UCB units.

Said pooled UCB units, or fraction thereof containing cells, obtained at the end of the method is preferably stored at a temperature below -70 °C, preferably below - 80 °C, more preferably in liquid nitrogen.

It is further described a method for producing a population of expanded and activated NK cells from n UCB units, wherein:
- each UCB unit is preliminary diluted in a suitable culture medium, preferably in a RPMI medium before use; and/or
- after the red-cell/ erythrocytes depletion of each UCB unit or of the pooled n UCB units, the collected cells are resuspended in a suitable culture medium, preferably in a RMPI medium, or in medium type X-VIVO™ (Lonza), AIM-V™ medium (Invitrogen) or CellGro™ (CellGenix), this medium optionally containing fetal bovine serum AB negative (FBS); and/or
- if the collected cells from each red-cell depleted UCB unit or from the pooled red-cell depleted UCB units are stored frozen, the collected cells are resuspended in a suitable culture comprising a white cells cryoprotectant..

It is also described a method for producing a population of expanded and activated NK cells from n UCB units, wherein the ratio between the NK cells and the accessory cells present in the suitable medium for NK cells expansion/activation is comprised between 0.01 and 2, preferably between 0.05 and 1.0, more preferably between 0,1 and 0.5.

It is further described a method for producing a population of expanded and activated NK cells from n UCB units, wherein the accessory cells present in the suitable medium for NK cells expansion/activation and the NK cells to be expanded/activated are HLA-KIR mismatched.

It is further described a method for producing a population of expanded and activated NK cells from n UCB units, said method further comprising a step of CD56+ NK cells enrichment.

It is further described a method for producing for the production of at least two distinct pools a population of expanded, optionally, activated NK cells from UCB units, wherein the major HLA class I group recognized by NK cells for each pooled n UCB is different, and wherein each pool of a population of expanded, optionally, activated NK cells from n UCB units is produced by a method for producing a pooled activated and/or expanded NK cells according to the present invention.

It is also described a method for the production of at least 2, 3, preferably 4 distinct pools a population of expanded, optionally, activated NK cells from UCB units according to the present invention,, wherein the major HLA class I group recognized by NK cells for each pooled n UCB is different and selected from the group consisting of HLA A3/A11 which is recognized by KIR3DL2, HLA Bw4, which recognized by KIR3DL1, HLA C group 1 which is recognized by KIR2DL2/3 and HLA C group 2 which is recognized by KIR2DL2.

It is further described a a population of cells:
- obtained by the method as described above, or
- obtainable by the method as described above and wherein said "obtainable" population of cells contains cells, preferably NK cells originated from at least n UCB units, or fraction thereof containing NK cells, with n ≥ 2, preferably 2 < n ≤ 100 or 3 ≤ n ≤ 50, more preferably 3 ≤ n ≤ 25, and, preferably, wherein said n UCB units further present the same pattern for major HLA class I groups genotype, preferably wherein the major HLA class I group recognized by NK cells for each pooled n UCB is different and selected from the group consisting of HLA A3/A11 which is recognized by KIR3DL2, HLA Bw4, which recognized by KIR3DL1, HLA C group 1 which is recognized by KIR2DL2/3 and HLA C group 2 which is recognized by KIR2DL2.

It is also described a method for producing a population of expanded and activated NK cells from n UCB units, wherein said population of cells obtainable by the above method further exhibiting for each pooled n UCB a miss expression of one of the KIRs selected from the group of KIR2DL2 and KIR2DL3, KIR2DL1, KIR3DL1 and KIR3DL2.

It is further described a pharmaceutical composition comprising a population of pooled and activated and/or expanded cells, particularly NK cells, obtained or obtainable by the method according to the present invention.

It is also escribed a pharmaceutical composition comprising a population of pooled and activated and/or expanded cells, particularly NK cells, obtained or obtainable by the method according to the present invention for a use as drug.

It is further described a pharmaceutical composition according to the present invention further comprising a pharmaceutically acceptable carrier.

In the present description, "pharmaceutically acceptable carrier" refers to a compound or a combination of compounds made part of a pharmaceutical composition that do not cause secondary reactions and that, for example, facilitate the administration of the active compounds, increase their lifespan and/or effectiveness in the body, increase their solubility in solution or improve their preservation. Said pharmaceutically acceptable carriers are well known and will be adapted by those persons skilled in the art according to the nature and the mode of administration of the active compounds selected.

It is also described a method for a collection of storage containers for mammalian cells, preferably for human cells, wherein each of said storage containers contains a fraction of a production lot of a population of cells obtainable or obtained by the method according to the present invention.

It is further described that said collection of storage containers for mammalian cells contains expanded and/or activated NK cells.

It is also described that said collection of storage containers for mammalian cells or said composition, contains at least 10⁷, preferably 2 to 10. 10⁷ or 10 to 100. 10⁷ activated and/or expanded NK cells, depending of the weight of patient to be treated.

It is further described a method for that said storage containers collection or said composition contains NK cells and being essentially free of CD3+ T cells , preferably less than 0.1 % or less than 0.01 %,.

It is also described that said collection of storage containers for mammalian cells according to the present invention or said composition according to the present invention, contains:
- at least 75 %, preferably over 85 or 90 % of NK cells exhibiting the marker the marker CD56+ ;and/or
- at least 75 %, preferably over 80 % of NK cells exhibiting CD45RAdim..

It is further described a storage container of a collection of storage containers, or said composition, for its use for suppressing the proliferation of tumor cells, preferably for the prevention and/or the treatment of cancer or for the treatment of infection.

It is also described that said tumor cells or cancer to be treated are selected from the group of hematologic malignancy tumor cells, solid tumor cells or carcinoma cells, preferably leukemia cells, acute T cell leukemia cells, chronic myeloid lymphoma (CML) cells, acute myelogenous leukemia cells, chronic myelogenous leukemia (CML) cells, multiple myeloma cells, or lung, colon, prostate, glyoblastoma cancer.

It is further described that the pooled activated and/or expanded NK cells as prepared according to the invention or said composition may also useful for the treatment of infectious diseases or dysimmune/autoimmune diseases.

Finally, It is also described that the t cells contained in the storage container or the composition according to the present invention, are administered to the subject by a systemic or local route, depending of the disease/pathology to be treated. Preferably, said compounds may be administered systemically by intramuscular, intradermal, intraperitoneal or subcutaneous route, or by oral route. The composition comprising the antibodies according to the invention may be administered in several doses, spread out over time.

Their optimal modes of administration, dosing schedules and galenic forms may be determined according to criteria generally considered in the establishment of a treatment adapted to a patient such as, for example, the age or the weight of the patient, the seriousness of the patient's general health, tolerance to the treatment and side effects noted.

The invention will be further illustrated by the following figures and examples. However, these examples and figures should not be interpreted in any way as limiting the scope of the present invention.

### Description of figures:

**Figures 1-1 to 1-3** (sub-figures1, 2 and 3 of Figure 1) is a schema illustrating an example of a manufacture process of the present invention
**Figures 2 and 3** illustrate the NK proliferation obtained after or without CD3 depletion
**Figure 4** illustrates the NK proliferation obtained from pooled CD3-depleted UCB units
**Figure 5** illustrates the NK proliferation obtained from 5 pooled CD3-depleted UCB units
**Figure 6** illustrates the NK proliferation obtained from pooled UCB units without prior CD3-depletion
**Figure 7** illustrates the NK proliferation from pooled UCB units after 9 days of culture with CD3-non depleted UCBs
**Figure 8** illustrates the NK proliferation amplification factor obtained with 2 KIR-HLA matched UCBs and amplified with PLH accessory cells
**Figure 9** illustrates the NK proliferation amplification factor obtained with 2 KIR-HLA mis and matched UCBs amplified with PLH accessory cells.

### EXAMPLE 1: Materials and Methods

### A) Cells:

### PLH (Example 4): no HLA-C1, ECACC bank n°88052047, IHW number 9047

This cell line was obtained by EBV immortalization of B lymphocytes coming from a scandinavian woman. This cell is completely HLA genotyped and have the particularity to express HLA Class I alleles from C group 2, A3/A11 and Bw4 types but not from C group 1 (complete informations on IMGT/HLA database).

This cell line is used as accessory cell for NK amplification/activation protocol because it allows to choose a specific HLA mismatch between accessory cell and UCBs (expressing HLA C group 1, and potentially the associated inhibitory receptor KIR2DL2/3). Being transformed by EBV infection increases its NK activation ability because of membranary expression of some viral induced ligands for NK activating receptors.

### HOM-2 (Example 4): no HLA-C2, ID n°HC107505, IHW number 9005

This cell line was obtained by EBV immortalization of B lymphocytes coming from a Canadian/North American woman. This cell is completely HLA genotyped and have the particularity to express HLA Class I alleles from C group 1, A3/A11 and Bw4 types but not from C group 2 (complete informations on IMGT/HLA database).

This cell line is used as accessory cell for NK amplification/activation protocol because it allows to choose a specific HLA mismatch between accessory cell and UCBs (expressing HLA C group 2, and potentially the associated inhibitory receptor KIR2DL1). Being transformed by EBV infection increases its NK activation ability because of membrane expression of some viral induced ligands for NK activating receptors.

### B) Media, buffers and cytokines:

1/Density gradient cell separation medium of Ficoll and sodium diatrizoate used for the separation of lymphocytes: Histopaque-1077 from Sigma Aldrich, Saint Louis, MO, USA
2/Kit for counting cells and looking at their viability with the Muse machine, labelling the cells with 7AAD and a fluorescent DNA probe: count and viability kit from Millipore, Darmstadt, Germany
3/Cellular culture medium: RPMI 1640 Glutamax from Invitrogen, Carlsbad, CA, USA, purchased from France distributor Thermo Fisher Scientific
4/Nutrient source in cellular culture medium: Fœtal Bovine Serum from Invitrogen, Carlsbad, CA, USA, purchased from France distributor Thermo Fisher Scientific
5/Organic solvent for cells freezing: dimethyl sulfoxyde, DMSO from B. Braun, Melsungen, Germany
6/Buffer for flow cytometry labelling: PBS from Invitrogen, Carlsbad, CA, USA, purchased from France distributor Thermo Fisher Scientific
7/Cytokine for NK amplification/activation: recombinant human rhIL-2 from ebioscience, San Diego, CA, USA
8/Cytokine for NK amplification/activation: recombinant human rh-IL15 from Miltenyi, Bergisch Gladbach, Germany

### EXAMPLE 2: Example of manufacturing process

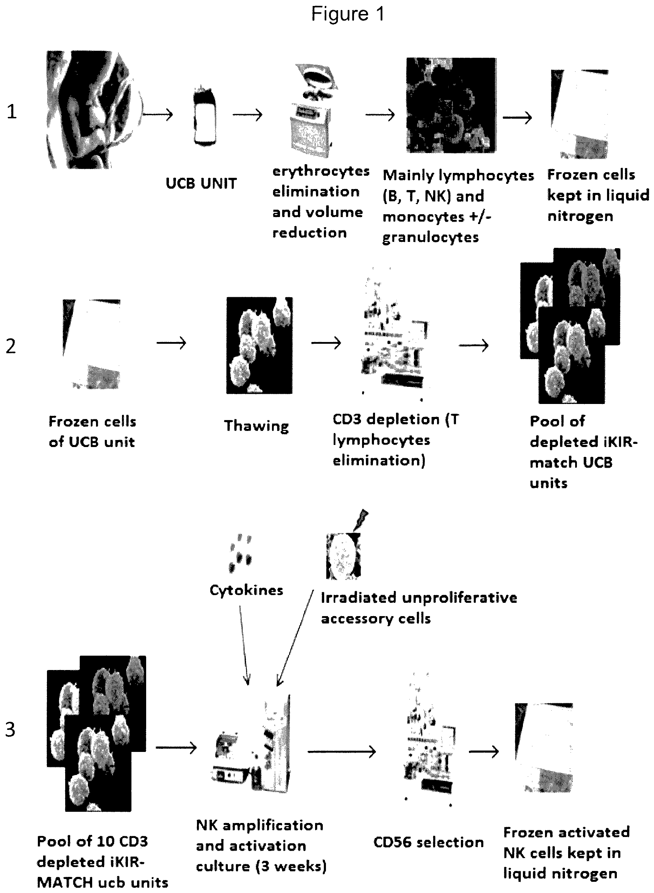

### Process details: see Figures 1-1 to 1-3

- UCBs were processed by ficoll UCB mononuclear cells isolation before first freezing.
- CD3 depletions were done with a manual magnetic depletion kit.
- Pooled UCBs present the same pattern for major HLA class I groups genotype (each HLA group is recognized by a major inhibitory KIR by NK cells): HLA A3/A11, recognized by KIR3DL2; HLA Bw4, recognized by KIR3DL1; HLA C group 1, recognized by KIR2DL2/3; HLA C group 2 recognized by KIR2DL1.
- Pooled UCBs are activated with an accessory cell missing one of the HLA recognized by the expressed pooled UCBs iKIRs.
- NK cells were amplified for 20-24 days.
- Cytokines used are IL-2 (100IU/ml) and IL-15 (5ng/ml). These concentrations can be modified to obtain similar results.
- Accessory cells are EBV-immortalized cell lines (cells expressing virus induced activating ligands) with specific HLA genotypes (one major HLA class I group missing).
- Accessory cells can be irradiated by different ways with different irradiation doses (here we mainly used 20 seconds UV irradiation, but also 105Gy gamma irradiation for the last experiment, that showed better amplification results).
- Irradiated accessory cells can be used with or without prior cryopreservation: freshly irradiated cells or as irradiated cryopreserved cells (irradiation just before freezing).
- For the 3 last experiments, irradiated accessory cells were added to the UCB cells at NK:accessory cell ratio 1:4, each 3-4 days (days 0;4;8;12;+/-15;+/-18). Some results (previous and other not shown results) were obtained using ratio 1:2, or ratio total cells:accessory cells from 1:1 to 1:3, with addition frequencies from 3 days to 7 days. This parameter can be changed, still obtaining similar amplification/activation results.
- In the experiments shown we didn't perform the CD56+ selection at the end of the process because NK cells derived from pooled CD3-depleted UCBs represented already more than 90% of alive cells at the end of the process. The CD56 selection step is not essential, but will probably improve NK purity and be preferable (and potentially totally required) for a pharmaceutical product.

Some steps of the process can be changed:
- UCBs will be processed differently before first freezing, using a GMP-compliant method such as Hetastarch™ or PrepaCyte CB™ device (or other existing and clinically accepted method).
- Even if current preclinical and clinical knowledge show that a iKIR-HLA mismatch gives better results than iKIR-HLA match, it is still possible that in our case iKIR-HLA has different influence in clinical outcome. So for the moment, the literature knowledge-based development should be with a process using NK/accessory cell mismatch and NK/patient same mismatch. Future preclinical and clinical data could change this parameter if unnecessary.

- NK amplification culture duration can be optimized: from 14 to 28 days.
- IL-2 and IL-15 concentrations can be optimized.
- The CD3-depletion will be done with an automatic clinically accepted device such as cliniMACS.
- The CD3-depletion can also be done just after erythrocyte elimination and volume reduction (maybe better results in term of NK recovery).
- One of the results demonstrates that in some undefined cases, the CD3-depletion is not necessary for UCB pooled NK cells good amplification/activation.
- To obtain an important quantity of activated multi-donors-derived NK cells characterized in a unique pharmaceutically defined lot, the preferentially CD3-depleted UCB units can be pooled at various moments of the process: before amplification culture, during amplification culture, or at the end of the amplification culture.

### EXAMPLE 3 : OBJECTIVES

### 1. First experiment

Because it is known that T lymphocytes from different donors will kill each other by HLA differences recognition, and because NK cells need activator signal to be cytotoxic, we asked whether it is possible to pool CD3-depleted UCBs expressing the same major HLA groups (depending their recognition by inhibitory KIR's) but not the same HLA alleles. Total mononuclear cells and CD3-depleted mononuclear cells from 3 UCBs were pooled to verify if CD3-depletion was essential.

### 2. Second experiment

Because we want to produce 4 class of NK cells presenting an iKIR-HLA mismatch for each major iKIR/HLA pair, we needed to investigate if success of pooling UCBs was only due to the first particular HLA genotyping used previously or could be reproduced with another HLA genotyping of UCBs: We asked whether another accessory cell line using another iKIR-HLA mismatch will allow NK amplification/activation from a pool of 3 CD3-depleted UCBs expressing the same HLA groups.

### 3. Third experiment

Because to treat around 100 patients we will need to pool 10 UCBs, we asked whether a pool of 5 UCBs (half) expressing the same HLA groups allow the same NK amplification/activation.

### EXAMPLE 4: Experiments carried out

### 1. First experiment

UCB mononuclear cells obtained by Ficoll separation were cryopreserved, then thawed and CD3-depleted using a stem cell kit for a part. Three CD3-depleted or total UCBs with same the major HLA class 1 groups A3/A11+,Bw4+,C1+,C2+ genotype were pooled and cultured for 21-25 days with IL-2, IL-15 and irradiated accessory cells PLH (A3/A1 1+,Bw4+,C1-,C2+ genotype) added each 4 days.

### 2. Second experiment

UCB mononuclear cells obtained by Ficoll separation were cryopreserved, then thawed and CD3-depleted using a stem cell kit. Three CD3-depleted UCBs with same the major HLA class 1 groups A3/A11-,Bw4+,C1-,C2+ genotype were pooled and cultured for 21-25 days with IL-2, IL-15 and irradiated accessory cells HOM-2 (A3/A11+,Bw4+,C1+,C2-genotype) added each 4 days.

### 3. Third experiment

UCB mononuclear cells obtained by Ficoll separation were cryopreserved, then thawed and CD3-depleted using a stem cell kit. Five CD3-depleted UCBs with same the major HLA class 1 groups A3/A1 1-,Bw4+,C1+,C2- genotype were pooled and cultured for 21 days with IL-2, IL-15 and irradiated accessory cells PLH (A3/A11+,Bw4+,C1-,C2+ genotype) added each 4 days.

### 4. Evaluated parameters

Alive NK cells were regularly counted using the MUSE Millipore system and flow cytometry characterization of cellular composition in the culture.

Expression of activating markers of NK cells was regularly evaluated by flow cytometry (CD16 for potent synergistic effect with monoclonal antibody therapies; CD69 as common activating receptor).

At day 20 of culture, cytotoxicity was evaluated against well-known K562 target cells, and tumoral cells for experiment 2 and 3 (2h incubation with NK:K562 ratio 3:1, NK:purified B lymphoma cells ratio 3:1, NK:AML cells (in total PBMC sample of the patient) ratio 10:1).

### EXAMPLE 5: RESULTS

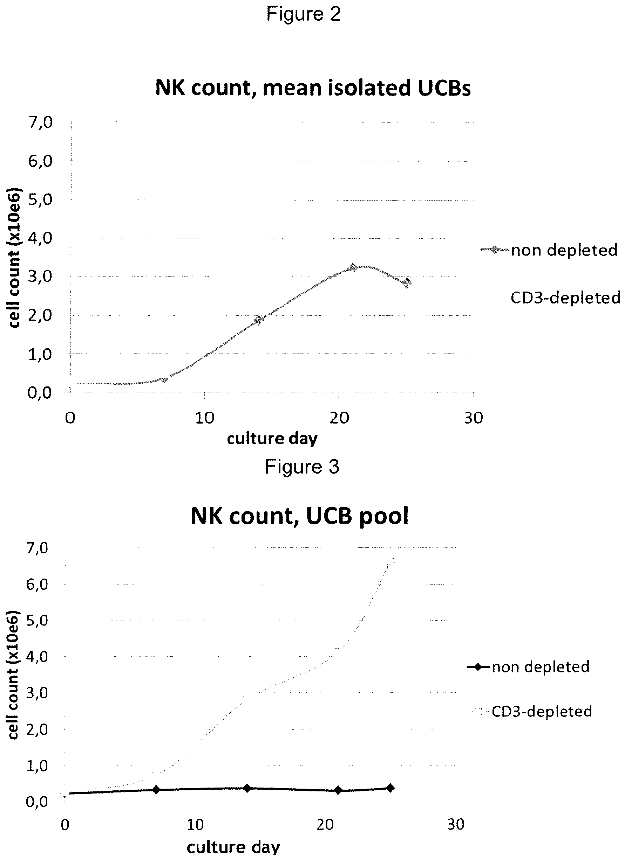

### 1. First experiment (see figures 2 and 3)

UCB 1: HLA A11:01/A29:02, B35:01/B44:02, C04:01/C16/01 > HLA A3/A11+, Bw4+, C1+, C2+
UCB2: HLA A11:01/A23:01, B35:02/B49:01, C04:01/07:01 > HLA A3/A11+, Bw4+, C1+, C2+
UCB3: HLA A2/A3, B18/B51, C5/C14 > HLA A3/A11+, Bw4+, C1+, C2+

NK proliferation from isolated UCBs show better results after CD3-depletion because T lymphocytes are in competition with NK cells for proliferation with the cytokines used (and CD8-T lymphocytes directed against EBV antigen are also stimulated by accessory cells).

NK from pooled CD3-depleted UCBs proliferate similarly than from isolated UCBs, but if UCBs are not CD3-depleted, T lymphocytes from the different donors are cytotoxic for the other one and NK cells cannot proliferate.

**Table 1:**

| | UCB 1 | UCB 2 | UCB 3 | pooled UCBs | CD3-depleted UCB 1 | CD3-depleted UCB 2 | CD3-depleted UCB 3 | pooled CD3-depleted UCBs |
|---|---|---|---|---|---|---|---|---|
| NK amplification factor | 2,6 | 17,8 | 15,7 | 1,6 | 20 | 14,9 | 76,7 | 23,9 |
| % NK CD16+ (ADCC-related) | 72,7 | 80,2 | 72,9 | 46 | 54,4 | 63,6 | 63,6 | 68,3 |
| % NK CD69+ | 86,7 | 88,6 | 94,7 | 94,3 | 92,9 | 95,1 | 96 | 86,6 |
| common target lysis % | ND | ND | ND | ND | 64,1 | 58 | 50,9 | 52,7 |

NK amplification factor is relatively low in this experiment due to technical issue.

Activating receptors are well expressed, and cytotoxicity against common target K562 of cultured NK cells is highly better than with un-activated NK cells.

This experiment showed that pooling UCB with same major HLA groups genotyping for NK amplification is feasible but require prior CD3-depletion. Amplified NK cells are well-activated.

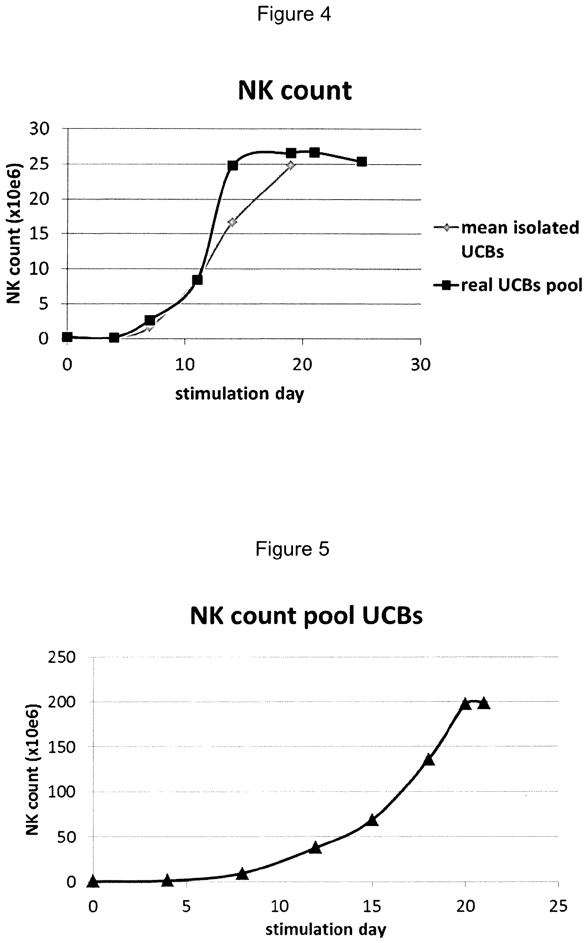

### 2. Second experiment (see Figure 4)

UCB1: HLA A01/02, B27:05/B40:02/C02:02/C15:02 > HLA A3/A11-, Bw4+, C1-, C2+
UCB2: HLA A2/A31, B50/B51, C06:02/15:02 > HLA A3/A11-, Bw4+, C1-, C2+
UCB3: HLA A23/A24, B44/B44, C4/C5 > HLA A3/A11-, Bw4+, C1-, C2+

NK proliferation from pooled CD3-depleted UCBs with this new genotype is similar to NK proliferation with isolated CD3-depleted UCBs.

**Table 2:**

| | CD3-depleted UCB 1 | CD3-depleted UCB 2 | CD3-depleted UCB 3 | pooled CD3-depleted UCBs |
|---|---|---|---|---|
| NK amplification factor | 86,3 | 184,4 | 47,1 | 124,7 |
| % NK CD16+ (ADCC-related) | 86,3 | 81,6 | 99,8 | 90 |
| % NK CD69+ | 99,6 | 94,9 | 99,2 | 98,5 |
| common target lysis % | 93 | 97,6 | 90,1 | 87,7 |
| B lymphoma tumoral cells lysis % | 37 | 48,2 | 78,4 | 31,6 |

NK amplification factor is higher in this experiment (no technical issue), but can still be improved by protocol optimization specifically for the new accessory cell line.

Activating receptors are very well expressed. Cytotoxicity against common target K562 of cultured NK cells is highly better than with unactivated NK cells, and we observe a significant cytotoxicity against B lymphoma tumoral cells with a 2 hour incubation.

Pooling CD3-depleted UCBs with another major HLA groups genotype, and amplifiying NK cells with another iKIR-HLA mismatch and another accessory cell line is feasible. Amplified NK cells are well-activated.

### 3. Third experiment (see Figure 5)

UCBs : HLA A3/A11-, Bw4+, C1+, C2-

**Table 3:**

| | pooled CD3-depleted UCBs |
|---|---|
| NK amplification factor | 583,2 |
| % NK CD16+ (ADCC-related) | 81,7 |
| % NK CD69+ | 99,8 |
| common target lysis % | 97,9 |
| AML tumoral cells lysis % | 10,4 |

| | |
|---|---|
| NK proliferation from 5 pooled CD3-depleted UCBs is good. | |

NK amplification factor is higher in this experiment.

Activating receptors are very well expressed. Cytotoxicity against common target K562 of cultured NK cells is highly better than with unactivated NK cells, and we observe a small specific cytotoxicity against AML tumoral cells with a 2 hour incubation (but we could'nt observe cytotoxicity after 20h because at this time patient cells died because of thawing).

Pooling 5 CD3-depleted UCBs and amplifying NK cells with an important amplification factor is feasible with our manufacturing process. Amplified NK cells are well-activated.

### 4. Complementary results

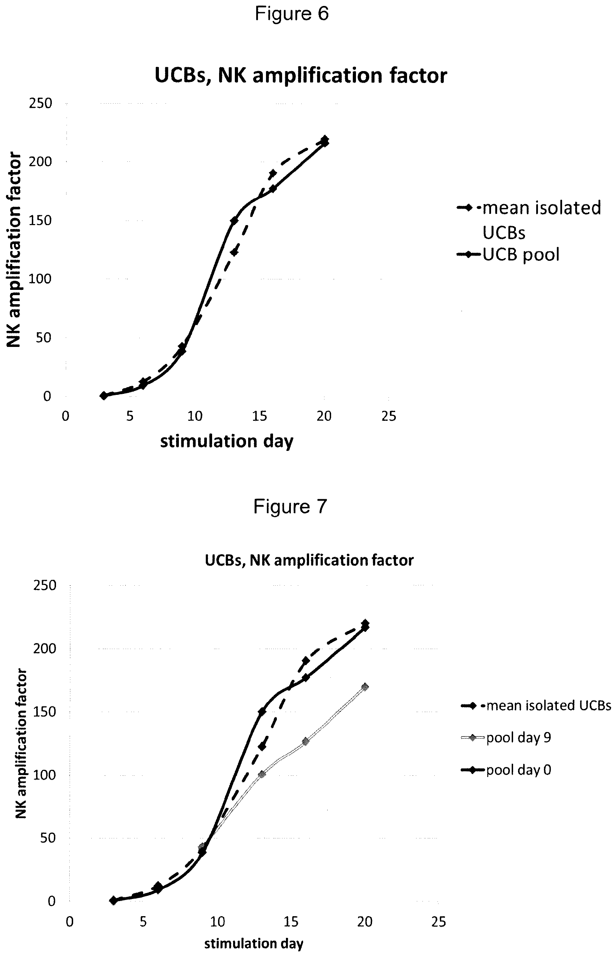

- Experiment showing good amplification of NK cells from pooled UCBs without prior CD3-depletion (no reproducibility assay): (see Figure 6)
   3 iKIR-HLA mismatch UCBs amplified with PLH:
   UCB 1: HLA A2:01/A68:01; B38:01/B57:01; C6:02/C12:03 > C1+, C2+, A3/A11-, Bw4+
   UCB 2: HLA A1:01/A2:01; B52:01/B57:01; C6:02/C12:02 > C1+, C2+, A3/A11-, Bw4+
   UCB 3: HLA A02/02; B15:09/B50:02; C06/C07 > C1+, C2+, A3/A11-, Bw4-

NK amplification can be similar in isolated or pooled UCBs without prior CD3-depletion.
- Experiments showing possibility of pooling after 9 days culture (with CD3-non depleted UCBs):

### 1/ same previous experiment (see Figure 7)

**Table 4:**

| | UCB 1 | UCB 2 | pool D0 | pool D9 |
|---|---|---|---|---|
| % B lymphoma lysis | 74 | 91 | 90 | 91 |

It is possible to pool 9 days activated NK cells (here without prior CD3-depletion) keeping a significant but lower NK amplification.

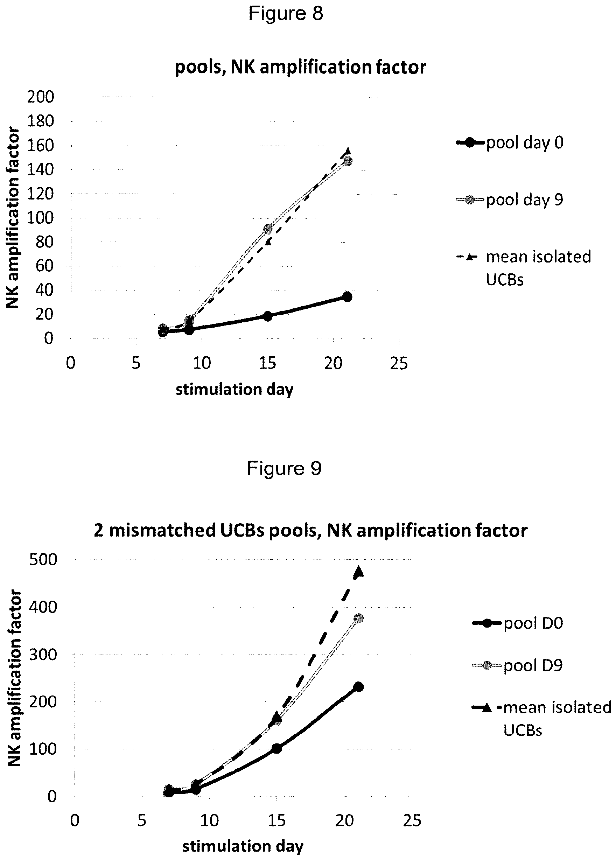

### 2/ Experiment with 2 iKIR-HLA matched UCBs amplified with PLH: (see Figure 8)

UCB 1: HLA A11:01/A29:02, B35:01/B44:02, C04:01/C16/01 > HLA A3/A11+, Bw4+, C1+, C2+
UCB2: HLA A11:01/A23:01, B35:02/B49:01, C04:01/07:01 > HLA A3/A11+, Bw4+, C1+, C2+

When NK didn't amplify properly in CD3-non depleted, pooling UCBs after 9 days amplification (increasing NK% and NK activation status, but still with high T lymphocytes %) seemed to overcome the problem. They showed an in vitro similar good cytotoxicity against B lymphoma tumoral cells (overnight, ratio E:T 1:1).

### 3/ Experiment with 2 iKIR-HLA mismatched UCBs amplified with PLH: (see Figure 9)

UCB1: HLA A02:02/30:01, B42:01/B53:01, C04:01/17:01 > HLA A3/A11-, Bw4+, C1-, C2+
UCB2: HLA A11:01/A23:01, B35:02/B49:01, C04:01/07:01 > HLA A3/A11+, Bw4+, C1+, C2+

**Table 5:**

| | UCB 1 | UCB 2 | pool D0 | pool D9 |
|---|---|---|---|---|
| % B lymphoma lysis | 74 | 92 | 96 | 97 |

NK cells from CD3-non depleted iKIR-HLA mismatched pooled UCBs showed a lower amplification factor, and pooling these UCBs after 9 days amplification gave better NK amplification. They showed an in vitro similar good cytotoxicity against B lymphoma tumoral cells (overnight, ratio E:T 1:1).

### EXAMPLE 6: PERSPECTIVES

### 1. Process optimization

Preferably, the manufacturing process of pooled activated/expanded NK cells according to the present invention will be adapted to the pharmaceutical regulatory obligations, and every step of the process adapted for the best quality guarantee.
- First, and for example, acceptance criteria of UCB units must be set, such as more than 1.4 or 1.6 10⁶ total nucleated cells (currently 1.85 10⁶ total nucleated cells for our local UCB bank), with potentially a minimal threshold for the NK percentage such as 7% (3-15% NK generally observed in UCB total nucleated cells).
- The "Ficoll" method used in the above examples for UCB mononuclear cells (UCBMC) isolation can be easily replaced by well-adapted standard and well-known method for clinical application, and pharmaceutical conditions, for example using a closed sterile single use system with bags, using adapted procedures such as HES 6% and centrifugations erythrocytes elimination and volume reduction, or Prepacyte CB isolation system. These systems certainly improve the total nucleted cell recovery in the first step.
- Preferably, CD3-depletion of UCBMCs can be better adapted to regulatory compliances and/ or GMP process for pharmaceutical uses, for example with an adapted clinically upgradable material such as CliniMACS™, and by determining the best step time for CD3-depletion whether it is needed, before or after first cryopreservation step for the best cell recovery and the best CD3-depletion quality.
- Preferably, the freezing, cryopreservation and thawing procedures for UCBMC can be improved using authorized procedures for clinical applications after validation of the manufacturing process. Adapted material for bag closed system can be used and cryopreservation conditions (media, cell concentration) can be easily optimized by the skilled person for the method of the present invention. These optimization steps only should certainly improve the total cell recovery after thawing. In the same time, the acceptance criteria for each thawed UCBMCs to go further into the manufacturing process according to pharmaceutical guidelines should be set.
- Preferably, HLA-genotyping and inhibitory KIR expression evaluation procedures should be validated to select the different UCB units allowed to be pooled for the amplification/activation step: selection criteria should be set for each lot.
- Preferably, GMP compliant upgradable accessory cells, whether they will be included in the method of the invention, with a final screening on NK amplification:activation for clones selection. Final accessory cells must be well-characterized for use in a therapeutic agent production procedure. This optimization step could also improve NK amplification/activation results.
- Preferably, irradiation procedure will be optimized and validated for the best amplification/activation results with clinically adapted quality parameters, and acceptance criteria of cryopreserved irradiated accessory cells lots will be set, including unproliferation evaluation, cells viability, EBV inactivation ...etc.
- Irradiated accessory cells exact addition procedure will be optimized for the final clones used in the process including accessory cells.
- Preferably, a dynamic culture closed system in bioreactors will be used for amplification/activation step with at least 5, preferably 10 pooled UCB units, such as the Wave system ™(GE Healthcare) already tested for NK culture.
- Preferably, culture medium used for the amplification/activation step, using animal serum-free media such as X-VIVO™ media from Lonza, CellGro SCGM™ from Cellgenix or AIM V ™ from Invitrogen (already tested for NK cultures) can be used..
- Preferably, CD56 positive selection of amplified/activated NK cells using an adapted clinically upgradable material such as CliniMACS™, will be used.

### 2. Pharmaceutical development: final product characterization and acceptance criteria

Preferably, a step of acceptance criteria of final amplified/activated products must will be included in the process, including product identification steps (genetic stability, chimerism, phenotype) and a standard potency evaluation procedure.
- Preferably, the genetic stability of NK cells before and after the process of the present invention will be be checked, looking at their karyotype (for example by G-banded karyotyping or cytoscanHD microarray methods wel-known by the sfilled person), and the chimerism of the final pooled NK cells from the different donors must be defined (for example by standard multiplex PCR STR methods).
- Preferably and to better identify and characterize the final product and to define acceptance criteria, the expression of more NK phenotypical markers (NKG2D, NKG2C, CD94, NKp44, NKp30, NKp46, CD158...) will be evaluated (for example by flow cytometry).
- Preferably, each product lot will be tested with a validated cytotoxicity assay against commonly used well-known target cells
- Preferably, the absence of contaminations such as bacteria, fungi, mycoplasma and viruses (particularly EBV) must be verified during or after the final step of the process, as the absence of endotoxins and cytokines used during the manufacturing process.

## Claims

1. A method for the production of a population of expanded activated NK cells, comprising:
(A) producing a population of cells containing NK cells from at least n UCB units, or fraction thereof containing said NK cells, by a method comprising the steps of:
(a) providing at least n umbilical cord blood units (UCB units), or fraction thereof containing said cells, with n ≥ 2, and
(b) pooling said at least n UCB units, or fraction thereof containing said cells, to produce the population of cells
(B) expanding and activating said NK cells obtained from the step (A) in a suitable medium to produce said population of expanded activated NK cells; and
(C) recovering said expanded activated NK cells,
wherein said method comprising in step (A),
- a step (c) of depleting T cells from said population of cells obtained in (b), or
- a step of depleting T cells contained in each of said n UCB units before the step (b) of pooling said UCB units,
wherein said n UCB units when pooled present the same pattern for major HLA class I groups genotypes and wherein said major HLA class I group is selected from the group consisting of HLA A3/A11 which is recognized by KIR3DL2, HLA Bw4 which is recognized by KIR3DL1, HLA C group 1 which is recognized by KIR2DL2/3, and HLA C group 2 which is recognized by KIR2DL; and
wherein the suitable medium to expand and to activate the NK cells in step (B) comprises accessory cells, said accessory cells and the NK cells to be expanded and activated being HLA-KIR mismatched.

2. The method of claim 1, wherein in step a), 3 ≤ n ≤ 50).

3. The method of claim 1, wherein said accessory cells are irradiated.

4. The method of claim 1, wherein said accessory cells are immortalized.

5. The method of claim 1, wherein each of said n UCB units or the pool of UCB units are red cells depleted.

6. The method of claim 1, wherein the UCB units used in step (A) are thawed UCB units from frozen stored UCB units.

## Patentansprüche

1. Verfahren für die Herstellung einer Population von vergrößerten aktivierten NK-Zellen, das Folgendes umfasst:
(A) Herstellen einer Population von Zellen, die NK-Zellen aus wenigstens n UCB-Einheiten oder einer Fraktion davon enthält, die die NK-Zellen enthält, durch ein Verfahren, das die folgenden Schritte umfasst:
(a) Bereitstellen von wenigstens n Nabelschnurbluteinheiten (*umbilical cord blood units* - UCB-Einheiten) oder einer Fraktion davon, die die Zellen enthält, mit n ≥ 2, und
(b) Poolen der wenigstens n UCB-Einheiten oder einer Fraktion davon, die die Zellen enthält, um die Population von Zellen herzustellen
(B) Vergrößern und Aktivieren der NK-Zellen, die aus dem Schritt (A) gewonnen wurden, in einem geeigneten Medium, um die Population von vergrößerten aktivierten NK-Zellen herzustellen; und
(C) Zurückgewinnen der vergrößerten aktivierten NK-Zellen, wobei das Verfahren in Schritt (A) Folgendes umfasst:
- einen Schritt (c) des Erschöpfens von T-Zellen aus der Population von Zellen, die in (b) gewonnen wurde, oder
- einen Schritt des Erschöpfens von T-Zellen, die in jeder der n UCB-Einheiten enthalten sind, vor dem Schritt (b) des Poolens der UCB-Einheiten, wobei die n UCB-Einheiten, wenn sie gepoolt sind, das gleiche Muster für Genotypen der Haupt-HLA-Klasse I-Gruppen darlegen und wobei die Haupt-HLA-Klasse I-Gruppe aus der Gruppe ausgewählt ist, die aus HLA A3/A11, das durch KIR3DL2 erkannt wird, HLA Bw4, das durch KIR3DL1 erkannt wird, HLA C-Gruppe 1, die durch KIR2DL2/3 erkannt wird, und HLA C-Gruppe 2, die durch KIR2DL erkannt wird, besteht; und wobei das geeignete Medium, um die NK-Zellen in Schritt (B) zu vergrößern und zu aktivieren, Hilfszellen umfasst, wobei die Hilfszellen und die zu vergrößernden und zu aktivierenden NK-Zellen HLA-KIR-fehlgepaart sind.

2. Verfahren nach Anspruch 1, wobei in Schritt a) 3≤n≤ 50) ist.

3. Verfahren nach Anspruch 1, wobei die Hilfszellen bestrahlt werden.

4. Verfahren nach Anspruch 1, wobei die Hilfszellen immortalisiert werden.

5. Verfahren nach Anspruch 1, wobei jede der n UCB-Einheiten oder der Pool von UCB-Einheiten von roten Blutkörperchen erschöpft sind.

6. Verfahren nach Anspruch 1, wobei die in Schritt (A) verwendeten UCB-Einheiten aufgetaute UCB-Einheiten aus gefrorenen gelagerten UCB-Einheiten sind.

## Revendications

1. Procédé de production d'une population de cellules NK activées expansées, comprenant :
(A) la production d'une population de cellules contenant des cellules NK à partir d'au moins n unités UCB, ou d'une fraction de celles-ci contenant lesdites cellules NK, par un procédé comprenant les étapes de :
(a) fourniture d'au moins n unités de sang de cordon ombilical (unités UCB), ou d'une fraction de celle-ci contenant lesdites cellules, avec n ≥ 2, et
(b) regroupement desdites au moins n unités UCB, ou d'une fraction de celles-ci contenant lesdites cellules, pour produire la population de cellules
(B) expansion et activation desdites cellules NK obtenues à partir de l'étape (A) dans un milieu approprié pour produire ladite population de cellules NK activées expansées ; et
(C) récupération desdites cellules NK activées expansées, ledit procédé comprenant à l'étape (A),
- une étape (c) de déplétion des cellules T de ladite population de cellules obtenue en (b), ou
- une étape de déplétion des cellules T contenues dans chacune desdites n unités UCB avant l'étape (b) de regroupement desdites unités UCB,
lesdites n unités UCB, lorsqu'elles sont regroupées, présentant le même modèle pour les génotypes majeurs des groupes HLA de classe I et ledit groupes HLA de classe I étant choisi dans le groupe constitué par HLA A3/A11 qui est reconnu par KIR3DL2, HLA Bw4 qui est reconnu par KIR3DL1, HLA C groupe 1 qui est reconnu par KIR2DL2/3, et HLA C groupe 2 qui est reconnu par KIR2DL ; et
le milieu approprié pour expanser et activer les cellules NK dans l'étape (B) comprenant des cellules accessoires, lesdites cellules accessoires et les cellules NK à expanser et à activer étant HLA-KIR mésapparié.

2. Procédé selon la revendication 1, dans lequel à l'étape a), 3 ≤ n ≤ 50).

3. Procédé selon la revendication 1, lesdites cellules accessoires étant irradiées.

4. Procédé selon la revendication 1, lesdites cellules accessoires étant immortalisées.

5. Procédé selon la revendication 1, chacune desdites n unités UCB ou le groupe d'unités UCB étant déplété en globules rouges.

6. Procédé selon la revendication 1, les unités UCB utilisées à l'étape (A) étant des unités UCB décongelées à partir d'unités UCB stockées congelées.
